# EUROPEAN PATENT APPLICATION

(11) **EP 1 405 618 A1**
(43) Date of publication of application: **07.04.2004**
(21) Application number: 02732768.3
(22) Date of filing: 22.05.2002
(51) Int. Cl.: A61F 5/44, A61F 5/453, A61F 5/455, A61G 9/00

(54) **SANITARY URINE COLLECTOR BAG**

(30) Priority: 23.05.2001 ES 200101322 U; 11.07.2001 ES 200101781 U
(71) Applicant: Gamez Cano, Matilde, 08032 Barcelona (ES)
(72) Inventor: Gamez Cano, Matilde, 08032 Barcelona (ES)
(74) Representative: Aragones Forner, Rafael Angel
(86) International application number: PCT/ES2002/000240
(87) International publication number: WO 2002/094142

(57) **Abstract**

The invention relates to a sanitary urine collector bag which fills a niche in the current market. According to the invention, normally appropriate urination jeans are not required by healthy or sick persons needing to urinate frequently, especially while on the move. The invention comprises a bag which is at least partially inserted inside the body, the opening of said bag being positioned internally to allow the urine to pass from the user or patient to the bottom of the bag. The opening, having part thereof inserted inside the body, forms a non-- return closing means which prevents the contents from inadvertently emptying. Additional closing jeans can be incorporated into the inventive bag. The internal part f the bag can be extended outwards, thereby forming a flattened tube with a secondary opening at the end thereof.

## Description

The invention has as its object sanitary urine collector bag.

The present invention is highly applicable for those persons having a need to urinate at relatively frequent intervals, especially when having to switch from one to another location.

Especially when travelling, healthy people having this need find themselves in serious difficulties when having to urinate since appropriate means are not available.

As for sick and bedridden people, for the evacuation of urine without using probes those elements being commonly called bedpans are used which correspond to flattened urinals frontally ending in the shape of a wedge or sharply acute angle, said bedpans being apt to collect the evacuated urine and faeces and to be used by persons of both sexes. Said bedpans have the drawback that even if they are very thoroughly cleaned the risk of contagion cannot be ruled out. In order to avoid this possibility disposable bags are arranged inside the bedpan in such a way that they are the elements finally being in contact with the organic waste and being later on deposited in special containers for their disposal.

For collecting the urine being evacuated by the sick and bedridden persons and especially in the case of the male sex bottles are used which are made of glass or the like and are transparent and thus allow to ascertain the colour and the approximate quantity of urine having been evacuated by the patient, said bottles being provided with a neck being apt to receive the male member. The possibility of ulterior contagion affecting other patients does also exist in this case even if the bottle is after its use submitted to a thorough and disinfectant cleaning.

There are at present several obstacles for the evacuation of urine, and thus for the healthy people being either stationed or on their way appropriate means allowing to urinate anywhere and at any time are not available, and for the sick and bedridden people the existing means present risks of contagion for the patients and their relatives and/or the members of the sanitary staff.

Said drawbacks have now been fully obviated by means of the sanitary bag being the object of the present invention, said bag allowing to collect the urine being anywhere and at any time evacuated by the healthy or sick person and to see the colour of the urine and the evacuated quantity, said bag being as well provided with nonreturn closure means preventing the undesired egress of the urine. Said bag is as easily available to the user as in the case of a tissue, since these bags can be supplied in an individually packaged arrangement or in a package containing several of them.

According to the present invention the urine collecting sanitary bag is essentially characterised in that it comprises an elongate bag being made of flexible, tough, waterproof and transparent plastics material, said bag being at least partially introduced within itself and thus having its opening arranged in the inside, said opening being at least partially open in order to thus allow the urine being evacuated by the patient to pass towards the bottom of the bag, said opening in combination with the inner walls of the introduced portion of the bag making up a by way of nonreturn closure means preventing the unintentional emptying of the bag, this latter being also possibly provided with additional closure means for its transportation.

According to the invention the opening can be totally or partially open, and in this latter case it can have been essentially provided with a punching having been provided by way of a punched line allowing to open it in order to thus adjust the urine passage, or else it can be provided with a micropunched stripe allowing the passage of the urine.

The closure means do likewise consist in an adhesive tape being fixed in the vicinity of the folded edge or inlet of the bag, said adhesive tape being originally protected by means of a releasable sheet before effecting the closure of the bag. The closure of the bag is preferably effected by twice folding on itself the area of the folded edge or inlet of the bag and by fixing the adhesive tape onto the opposite side of the bag.

The bag is provided with positioning means keeping it partially introduced within itself. Said positioning means can be weld means.

The present invention provides a second embodiment wherein the inner portion of the bag being partially introduced within itself extends towards the outside thus forming a flattened tube ending in a secondary opening.

According to the invention the bag is provided with a number of parallel streaks starting from the bottom, said streaks allowing to measure the quantity of urine having been evacuated by the patient besides viewing the colour of said urine.

According to the invention the weld is arranged in a transversal arrangement with respect to the main axis of the bag, in an area being arranged in the vicinity of the inlet or folded edge of the bag. The passage for the urine is provided at the central portion of the weld.

In the second embodiment the bag closure means are arranged on the outer tube next to the secondary opening.

These and other characteristics will be best made apparent by the following detailed description whose understanding will be made easier by the accompanying two sheets of drawings showing a practical embodiment cited only by way of an example not limiting the scope of the present invention.

In said drawings:
Figure 1 is an elevational view of the bag being the object of the present invention as per the preferred embodiment;
Figure 2 is a sectional view as per section line II-II in Figure 1;
   each of Figures 3a, 3b and 3c is a cross-sectional view as per section line III-III in Figure 1;
Figure 4 is a partially fragmented, sectional side elevation showing the bag being shown in Figure 2 and the positioning means for closing said bag;
Figure 5 is a perspective view of a second embodiment of the present invention;
Figure 6 is an elevational view of the second embodiment being shown in Figure 5, in its state being partially filled with urine and being placed in a normal position;
   each of Figures 7 and 8 is a fragmentary, sectional view as per section lines VII-VII and VIII-VIII in Figure 5;
Figure 9 is an elevational view of the bag of Figure 6, said bag being in this view partially filled with urine and placed in an inverted position, thereby showing the operation of the nonreturn closure means preventing the unintentional emptying of the bag; and
Figure 10 is a fragmentary, sectional view as per section line X-X in Figure 9 showing the operation of the nonreturn closure means.

According to the drawings the urine collecting sanitary bag being the object of the present invention consists in a bag being generally indicated at 1, said bag being made of flexible, tough, waterproof and preferably transparent material in order to properly visualise the quantity and colour of the urine being evacuated by the user or patient.

The bag of the invention being generally indicated at 1 is of elongated shape and is partially introduced within itself, and its opening E is arranged in the inside and spaced from the bottom F of the bag.

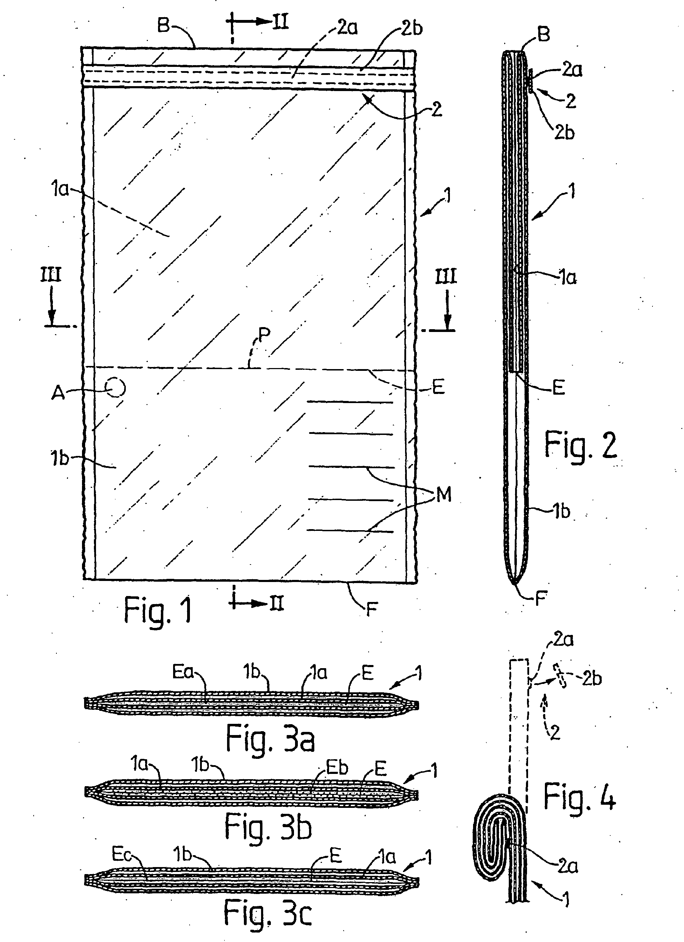

Figure 1 illustrates the preferred embodiment of the present invention.

According to the present invention the user or patient evacuates the urine through the inlet or folded edge B of the bag and through the opening E towards the bottom F of the bag 1. In combination with the inner walls 1a of the bag being introduced within itself the opening E makes up a bay way of nonreturn closure means preventing the unintentional emptying of the bag.

Said nonreturn closure means provide a tight and automatic closure of the bag 1.

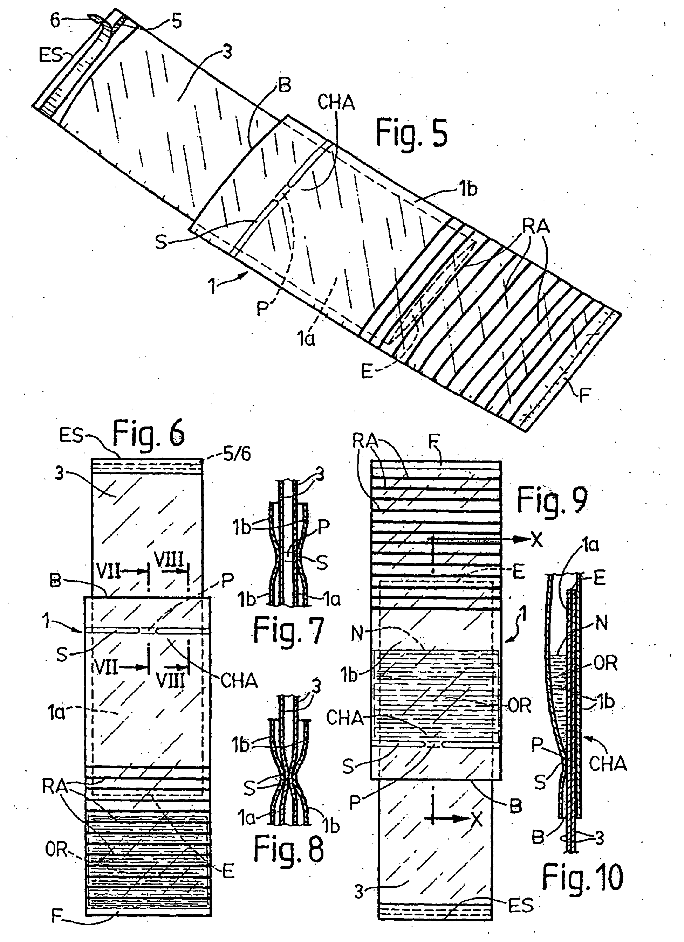

The bag is provided with additional closure means 2 on one of the outer face-sides of the bag 1, said additional closure means consisting in an adhesive tape 2a being originally protected by means of a releasable sheet 2b. As shown in detail in Figure 4, once the bag has been used by the patient/user having evacuated the corresponding urine through the inlet or folded edge B the protective sheet 2b having been originally provided is firstly removed, the corresponding end of the bag 1 being thereupon folded upon itself towards the side being opposite to the one where the adhesive tape 2a is arranged, one fold or preferably one double fold being possibly carried out for such a purpose, whereupon said adhesive tape 2a is then fixed against the opposite side of the bag.

The folded edge or inlet B defines an inner portion 1a of the bag and an outer portion 1b of said bag, the inner portion 1a of the bag comprising the opening E being through said inlet or folded edge B introduced in the inside of the outer portion 1b.

According to the present invention the opening E can be totally open, as shown at Ea in Figure 3a, in order to thus facilitate the use of the bag on the user's part, or it can be partially closed such as for example in form of a punching being provided by way of a punched line as shown at Eb in Figure 3b, said punched line allowing the user to tear open several punched junctions before using the bag in order to thus facilitate the evacuation, or in form of a micropunched stripe Ec being only diagrammatically shown in Figure 3c and through the micropunched or simply punched junctions being possibly arranged in any arrangement such as in a staggered arrangement or in any other arrangement allowing the urine to pass towards the bottom F of the bag through said opening through a by way of open passage P having been opened as per the user's wishes.

The arrangement of the inner portion 1a where the opening E can be totally or partially open provides a by way of nonreturn closure means having been described before and at least in part preventing the unintentional egress of urine or emptying of the bag, said egress or emptying being sealingly prevented by the additional closure means 2 in the position being illustrated in Figure 4, since when inverting the position of the bag 1 or when upsetting it said inner portion 1a of the bag 1 acts as a self-acting closure and thus to a large extent prevents the accidental emptying of the bag, said emptying being totally prevented when having arranged the additional closure means as has been said before. Without applying the additional closure means 2 in the arrangement Ea with a fully open opening the unintentional egress of the urine or emptying of the bag is of course more easily likely to happen, whereas this will be less likely to happen in the case of the arrangements Eb and Ec wherein the opening E is partially closed.

The invention provides the arrangement of a means for positioning the bag 1 in a position being partially introduced within itself, as illustrated in the Figures, thereby establishing the inlet or folded edge B and defining an outer part or portion 1a, as has been described. Said positioning means can be of any kind being properly apt to maintain the bag introduced within itself as shown in the Figures, and they can preferably be weld means being provided between the different sides and/or sheets forming the bag, said welds being preferably possibly arranged on both sides.

In order to display the quantity of urine having been evacuated by the patient the bag 1 being the object of the present invention has a number of marks M that can indicate the quantity of urine and can be, for example, marks corresponding to a quantity of 100, 200, 300, 400, 500, etc. cm³, as deemed convenient.

If such a feature is of interest, the bag 1 does as well comprise an area A being apt to allow to extract a sample by means of a syringe for its ulterior analysis.

The present invention provides a second embodiment being illustrated in Figures 5 through 10. In essence, the inner portion of the bag 1 being partially introduced within itself extends towards the outside thereby forming a flattened tube 3 ending in a secondary opening ES.

The bag 1 of this second embodiment comprises the bag itself being formed by the inner walls 1a and the outer walls 1b, and the outer extension forming the flattened tube 3. The flattened tube 3 projects from the inlet or folded edge B of the bag towards the outside in an extension of the inner walls 1a.

The bag 1 is defined by a linear heatseal S being arranged in a transversal arrangement with respect to the main axis of the bag 1 in an area being arranged in the vicinity of the inlet B of the bag, said linear heatseal being interrupted in a preferably central part and thus forming a passage P being apt to allow the urine OR being evacuated by the patient (see Figures 6, 9 and 10) to pass from the flattened tube 3 to the inside of the bag.

The bag 1 as per this second embodiment of the present invention has the precited nonreturn closure means CHA having been described before and in this embodiment corresponding to the above-mentioned passage P corresponding to an interruption of the weld S, and the inner walls 1a and the outer walls 1b of plastics material of the bag, these latter because of their nature being provided with static electricity maintaining said adjacent walls stuck together and thus closing the bag (see especially Figure 10 showing a sectional view in fragmentary detail). Said Figure allows to appreciate the level N of the urine OR in the bag 1 after having inverted the position of this latter with respect to the normal position of the bag as illustrated in Figure 6, and how the urine OR is maintained inside the bag 1 since the level N of the urine is below the opening E of the inner portion 1a of the bag 1, the flattened tube 3 being an extension of said inner portion 1a towards the outside.

But if the disposable bag 1 is in any way accidentally upset the static electricity inhabiting the inner and outer walls of the bag will keep them in full contact, and this combined with the very hydrostatic pressure exerted by the urine on the collecting bag contributes to maintaining said inner portions of the walls in full contact with each other; all this preventing the accidental egress of the urine OR being contained in the bag 1 because of any undesired, arbitrary upset, fall or accident.

Figure 6 illustrates the bag 1 in a vertical elevation with the urine OR on the bottom of the bag 1, this latter being provided with a number of parallel streaks RA like those of Figure 1 starting from the bottom F, said streaks allowing the members of the sanitary staff to measure the quantity of urine having been evacuated by the patient, as well as to view the colour of said urine.

The heatseal S can be of any convenient nature and can have any configuration and position, but preferably and as shown in the Figures it will be arranged in a transversal arrangement with respect to the main axis of the bag 1 and in an area being located in the vicinity of the inlet B of said bag.

The passage P being formed by means of an interruption of the above-mentioned linear heatseal S can likewise be arranged anywhere in the heatseal, but it will be preferably located at a central position as shown in the Figures.

As shown in the Figures, and especially in Figure 5, in an area being located in the vicinity of the secondary opening ES of the flattened tube 3 the bag 1 is provided with a closure means consisting in an adhesive strip 5 being arranged on one side of the tube and originally protected by a protective strip that will be removed in order to thus allow to close the disposable bag 1 with the adhesive strip 5 by folding the tube 3 upon itself.

The sanitary bag 1 being the object of the present invention is a disposable bag in order to thus avoid all contagion for the users/patients and the people taking care of them. The user (or his relatives/nursers) can keep in his pocket or handbag any number of bags in exactly the same way as in the case of the disposable tissues being conventionally used. Said bags are supplied in a conventional arrangement like the one being used in the case of the above-mentioned tissues, this allowing the user, whether healthy or sick (whether bedrid or not), to anywhere and at any time avail himself of the sanitary bag 1 irrespective of his being stationed or on his way or in a journey, etc.

The sanitary bag 1 being the object of the present invention provides an appropriate and available means to urinate anywhere and at any time and as many times as needed by the user, said means not having been available so far.

The bag of the invention can be formed as a one-piece bag, as shown in the Figures, or else it can comprise several sheet members being superimposed and welded to each other at both sides. If the bag is a one-piece bag it can be originally arranged as per a tubular or sheet-like configuration.

The bag of the present invention can have any convenient configuration and accessories such as the additional closure means 2 and 5/6 and others forming part of what makes up the sanitary bag of the invention.

## Claims

1. Sanitary urine collector bag of the type consisting in a urine collecting bag being provided with closure means; **characterised in that** it comprises an elongate bag (1) being made of flexible, tough, waterproof and transparent plastics material, said bag being at least partially introduced within itself and thus having its opening (E) arranged in the inside, said opening being at least partially open in order to thus allow the urine (OR) being evacuated by the patient to pass towards the bottom (F) of the bag, said opening (E) in combination with the electrostatically charged inner walls (1a) of the introduced portion of the bag making up a by way of nonreturn means to thus prevent the unintentional emptying of the bag, this latter being also possibly provided with closure means for facilitating its transportation.

2. A bag as per claim 1, **characterised in that** the opening (E) is at least partially open.

3. A bag as per claim 2, **characterised in that** the opening (E) is essentially provided with a punched line in order to thus adjust the urine passage.

4. A bag as per claim 2, **characterised in that** the opening (E) is provided with a micropunched stripe for the passage of the urine.

5. A bag as per claim 1, **characterised in that** the closure means (2, 5/6) consist in an adhesive tape (2a) being fixed in the vicinity of the inlet or folded edge (B) of the bag, said adhesive tape being originally protected by means of a releasable sheet (2b, 6).

6. A bag as per claim 5, **characterised in that** the closure of the bag is effected by folding it on itself and by fixing the adhesive tape (2a, 5) onto the opposite side of the bag (1) after having previously removed the releasable sheet.

7. A bag as per claim 1, **characterised in that** it is provided with positioning means (E, S) keeping the bag (1) partially introduced within itself.

8. A bag as per claim 7, **characterised in that** said positioning means consist in weld means (S).

9. A bag as per claim 1, **characterised in that** the inner portion of the bag (1) being partially introduced within itself extends towards the outside thus forming a flattened tube (3) ending in a secondary opening (ES).

10. A bag as per claims 1 and 9, **characterised in that** the bag (1) is provided with a number of parallel streaks (M, RA) starting from the bottom (F), said streaks allowing to measure the quantity of urine (OR) having been evacuated by the patient besides viewing the colour of said urine.

11. A bag as per claim 9, **characterised in that** the weld (S) is arranged in a transversal arrangement with respect to the main axis of the bag (1), in an area being arranged in the vicinity of the inlet (B) of the bag.

12. A bag as per claim 11, **characterised in that** the passage (P) for the urine (OR) is provided at the central portion of the weld (S).

13. A bag as per claim 10, **characterised in that** the bag closure means (5/6) are arranged on the tube (3) in an area being located in the vicinity of the outer secondary opening (ES).

14. A bag as per claims 1 and 9, **characterised in that** it is a disposable bag.
